# EUROPEAN PATENT SPECIFICATION

(11) **EP 4 615 553 B1**
(45) Date of publication and mention of the grant of the patent: **09.09.2026**
(21) Application number: 23887187.5
(22) Date of filing: 06.07.2023
(51) Int. Cl.: A61M 37/00, A61B 5/15, A61B 50/30, A61M 5/20, A61M 5/46

(54) **MICRONEEDLE PATCH DELIVERY APPLICATOR AND METHOD**
APPLIKATOR UND VERFAHREN ZUR ABGABE VON MIKRONADELPFLASTERN
APPLICATEUR D'ADMINISTRATION DE TIMBRE À MICRO-AIGUILLES ET MÉTHODE

(30) Priority: 08.11.2022 AU 2022903335
(43) Date of publication of application: 17.09.2025
(73) Proprietor: University of Southern Queensland, Toowoomba, Queensland 4350 (AU)
(72) Inventor: EBRAHIMINEJAD, Vahid, Toowoomba, Queensland 4350 (AU); FARAJI RAD, Zahra, Toowoomba, Queensland 4350 (AU)
(74) Representative: Wynne-Jones IP Limited
(86) International application number: PCT/AU2023/050626
(87) International publication number: WO 2024/098090

(56) References cited:
- EP-B1- 2 976 126
- WO-A1-2021/125955
- WO-A1-2021/125955
- JP-A- 2019 170 870
- JP-A- 2019 170 870
- US-A1- 2016 082 626
- US-A1- 2016 271 380
- US-A1- 2018 116 687
- US-A1- 2020 306 518
- US-A1- 2021 379 348
- US-B1- 6 537 242
- US-B2- 10 315 021
- US-B2- 10 716 926
- US-B2- 9 415 198
- EBRAHIMINEJAD VAHID ET AL: "Design, Development, and Testing of Polymeric Microblades: A Novel Design of Microneedles for Biomedical Applications", ADVANCED MATERIALS INTERFACES, vol. 9, no. 29, 7 September 2022 (2022-09-07), Weinheim, XP093326046, ISSN: 2196-7350, DOI: 10.1002/admi.202201115

## Description

### TECHNICAL FIELD

The present invention relates to an applicator and method of use for delivery of a microneedle patch to a surface of a subject.

### BACKGROUND

Drawing fluids from the body and introducing fluids and medicaments into the body have long been used for treating and diagnosing medical conditions, both in animals and human beings. Typically, these procedures are undertaken using a hypodermic needle or similar catheter arrangement.

Recent developments in microneedle patches has led to them increasingly becoming a viable and attractive alternative to conventional hypodermic needles. Such patches typically include a base layer or membrane with a single or an array of microneedles protruding from a common side.

Traditional hypodermic needles are invasive, stimulate pain, produce hazardous wastes and require trained medical staff for administration. In contrast, microneedle patches are minimally invasive devices which enable painless administration of therapeutic molecules and sampling of biomarkers, such as, e.g., interstitial fluid for point-of-care diagnostics, by bypassing the stratum corneum barrier of the skin.

However, in order to be an effective alternative to hypodermic needles, microneedle patches must be capable of penetrating skin layers deep enough to enable effective delivery of therapeutic agents or sampling of capillary blood or interstitial fluid, while avoiding stimulation of the underlying nerve system (Ebrahiminejad, V. *et al.,* 2022; Ma, G. & Wu, C., 2017). Furthermore, effective penetration must be uniformly achieved by all microneedles otherwise inadequate delivery or sampling of a volume of therapeutic agent or sample may result.

Moreover, the application of excessive or unequal force when applying a microneedle patch can cause failure of the patch before penetration and/or before effective delivery or sampling of a therapeutic agent or sample. For example, excessive or unequal force can cause breakage of the microneedles and/or buckling or bending of the patch.

The above challenges are further compounded by the skin's inherent elasticity, irregular surface and tendency to fold around microneedle projections.

For example, the "bed of nails" effect, whereby the outermost microneedles stretch the skin causing the inner microneedles to not, or only partially, penetrate the stretched skin, is commonly observed when applying a microneedle patch (Sausse Lhernould, M., *et al.* 2013).

In other examples, the skin's irregular surface and inherent elasticity imposes undesired lateral loads resulting in bending or buckling of the patch, possible breakage of the patch and, at a minimum, unpredictable microneedle array penetration (Badran, M. M., *et al.* (2009); Donnelly, R. F., *et al.* (2010)).

In view of the above, mechanical applicators have been developed that are capable of generating a controlled and uniform application force using a predetermined impact velocity and/or pressure collars or cones for pre-stretching the skin at an application site.

However, none of the prior art applicators provide an entirely satisfactory solution to the above challenges. Indeed, the present inventors have found such applicators to be overly complicated, limited to specific microneedle patches and/or incapable of adjustment for use with different skin types.

It will be clearly understood that, if a prior art publication is referred to herein, this reference does not constitute an admission that the publication forms part of the common general knowledge in the art in Australia or in any other country.

### SUMMARY OF INVENTION

Embodiments of the present invention provide an applicator and method of use for delivery of a microneedle patch to a surface of a subject, which may at least partially address one or more of the problems or deficiencies mentioned above or which may provide the public with a useful or commercial choice.

According to a first aspect of the present invention, there is provided an applicator for delivery of a microneedle patch to a surface of a subject, said applicator including:
a barrel having an open delivery end through which the microneedle patch is delivered to an underlying said surface of the subject;
an adjustable spring-loaded plunger slidable within the barrel for moving the microneedle patch through a delivery stroke towards the open delivery end;
a microneedle patch holder operatively associated with an end of the spring-loaded plunger for releasably holding the microneedle patch through the delivery stroke;
at least one leg pivotally coupled to a side of the barrel and configured to stretch taut said underlying said surface, said leg including a distal end for contacting the surface and a biasing mechanism for biasing the leg away from the barrel and thereby stretching the surface taut; and
an electric actuator operatively associated with the microneedle patch holder and configured to vibrate at least the microneedle patch holder at a selected frequency during the delivery stroke.

According to a second aspect of the present invention, there is provided an applicator for delivery of a microneedle patch to a surface of a subject, said applicator including:
a barrel having an open delivery end through which the microneedle patch is delivered to an underlying said surface of the subject;
a plunger mechanism including a plunger slidable within the barrel for moving the microneedle patch through a delivery stroke towards the open delivery end, an adjustable biasing mechanism associated with the plunger for biasing the plunger towards the open delivery end and a release member for releasing the plunger under a force of the biasing mechanism for movement through the delivery stroke;
a microneedle patch holder operatively associated with an end of the plunger for releasably holding the microneedle patch through the delivery stroke;
at least one leg pivotally coupled to a side of the barrel and configured to stretch taut said underlying said surface, said leg including a distal end for contacting the surface and a biasing mechanism for biasing the leg away from the barrel and thereby stretching the surface taut; and
an electric actuator operatively associated with the microneedle patch holder and configured to vibrate at least the microneedle patch holder at a selected frequency during the delivery stroke.

Advantageously, the applicator of the present invention through its combined use of an adjustable biasing mechanism, at least one leg for stretching taut the surface of the subject and the electric actuator provides an efficient means for effectively delivering a microneedle patch to a subject that achieves maximal penetration without stimulating underlying nerves. Specifically, the adjustable biasing mechanism, legs and electric actuator enable the applicator to efficiently deliver a microneedle patch regardless of the elasticity or irregular topology of the surface (i.e., skin).

As indicated above, the applicator of the present invention is for use in delivering a microneedle patch to a surface of a subject in a reliable and effective manner.

As used herein, the term "microneedle patch" may encompass any microscaled medical device used to administer a therapeutic agent or extract a biological sample.

Typically, the microneedle patch may include a generally planar support member and either a single or plurality of microneedles arranged thereon and extending from a common side of the support member.

The support member may include a pair of opposed surfaces interconnected by opposing edges. The opposed surfaces may include a needle protruding surface and an opposed outer surface.

The plurality of microneedles may be supported on and usually arranged in an array on the needle protruding surface of the support member.

The opposed outer surface may be configured to be releasably received on an applicator for delivery to the subject.

The microneedles may be solid, hollow, coated, dissolvable and/or hydrogel-forming.

In embodiments in which the microneedles are hollow, the microneedle patch may further include at least one reservoir in the support member and a channel providing fluid communication between at least one of the plurality of microneedles and the at least one reservoir for the delivery and/or extraction of a therapeutic agent or biological sample, respectively. The channel may extend along the at least one of the plurality of needles and may be an open or closed channel.

As used herein, the term "subject" may include plants and mammals, the latter especially including humans, primates, livestock animals, laboratory test animals, companion animals and wild animals (whether captive or free). Plants may include vascular plants (Embryophyta), especially crops, such as, e.g., food crops, horticulture, floriculture, and industrial crops. Livestock animals may include sheep, cattle, pigs, horses and donkeys. Laboratory test animals may include mice, rabbits, rats, pigs and guinea pigs. Companion animals may include dogs and cats.

As used herein, the term "surface" may include a skin of the subject, typically at least an outer layer of the skin of the subject.

As indicated, the applicator includes a barrel having an open delivery end through the microneedle patch is delivered to an underlying said surface of the subject, preferably a skin of the subject.

The barrel may be of any suitable size, shape and construction to at least partially house the plunger, the microneedle patch holder, and the electric actuator.

The barrel may be of unitary construction or may be formed/constructed from two or more barrel pieces.

The barrel may be formed from any suitable material or materials. Generally, the barrel may be formed from plastic and/or metal material or materials, preferably plastic.

Typically, the barrel may be formed from a mould, such as, e.g., compression moulding, injection moulding or blow moulding, preferably injection moulding. In such embodiments, the barrel may be formed from a polymeric material or materials.

The barrel may include the open delivery end, an opposed outer end and at least one sidewall extending therebetween, preferably longitudinally.

The at least one sidewall may be flat, rounded or curved.

The barrel may be tubular in shape.

The open delivery end may include a passageway defined therein for slidably receiving the plunger. The passageway may be centrally defined. The passageway may extend from the open delivery end at least partially towards the opposed outer end.

The barrel may have any suitable cross-sectional shape. For example, the barrel may have a circular, oval-shaped, triangular, rectangular, pentagonal, hexagonal or octagonal cross-sectional shape, preferably substantially circular.

The open delivery end and the passageway may be of any suitable size and shape for receiving the plunger, the microneedle patch holder and a microneedle patch therein.

The passageway may have a constant width or diameter extending from or near the open delivery end at least partially towards the opposed outer end.

In some embodiments, the open delivery end may be defined by an inwardly turned edge portion extending inwards from the open delivery end and extending at least partially around an inner periphery of the barrel at the open delivery end. Advantageously, the inwardly turned edge portion may prevent the plunger and the microneedle patch holder from over extending past the open delivery end at the end of the delivery stroke.

As indicated, the applicator includes a plunger slidable within the barrel for moving the microneedle patch through a delivery stroke towards the open delivery end.

The plunger may be of any suitable size, shape and construction.

The plunger may be of unitary construction or may be formed/constructed from two or more plunger pieces, preferably the latter.

The plunger may be formed from any suitable material or materials. Generally, the plunger may be formed from plastic and/or metal material or materials, preferably plastic.

Like the barrel, the plunger may typically be formed from a mould, such as, e.g., compression moulding, injection moulding or blow moulding, preferably injection moulding. In such embodiments, the plunger may be formed from a polymeric material or materials.

The plunger may include a pair of opposed ends and an elongate body extending therebetween, preferably in a linear direction.

The opposed ends may include a driving end and an opposed outer end.

The microneedle patch holder may extend from, or be mounted to, the driving end.

The opposed outer end may preferably extend through an opening defined in the outer end of the barrel so as to allow an operator to manually pull the plunger towards the outer end of the barrel after a delivery stroke, typically against a force of the spring or the biasing mechanism operatively associated with the plunger.

In some embodiments, the outer end of the plunger may include an operational grip to facilitate the operator in gripping and pulling the plunger.

The elongate body may be in the form of a plunger rod defined by at least one sidewall extending longitudinally between the opposed ends.

The plunger rod may have any suitable profile shape. For example, the plunger may have a circular, oval-shaped, triangular, rectangular, pentagonal, hexagonal or octagonal profile shape.

In use, the plunger may be slidable within the barrel between a retracted position and an extended position.

In the retracted position, the plunger may be retracted towards the outer end of the barrel against the force of the spring or the biasing mechanism, preferably manually retracted.

In the extended position, the driving end of the plunger (including the operatively associated microneedle patch holder) may be located at or near the open delivery end of the barrel.

As used herein, the term "delivery stroke" may refer to movement of the plunger from the retracted position to the extended position under the force of the spring or the biasing mechanism.

The biasing mechanism may include one or more springs, such as, e.g., coil or leaf springs. Of course, a person skilled in the art will appreciate that other types of biasing mechanisms, such as, e.g., magnets or magnetized elements and the like may be used.

In preferred embodiments, the biasing mechanism may include, or the plunger may be, a spring-loaded plunger. For example, the plunger may include a coil spring configured to sit about the plunger rod between the driving end and operatively associated microneedle patch holder and an inner surface of the outer end of the barrel. The coil spring may preferably be a compression spring.

In use, movement of the plunger to the retracted position may compress the spring and store mechanical energy configured to be released and drive the plunger through the delivery stroke.

In some embodiments, the plunger mechanism may further include a retaining member for retaining the plunger in the retracted position against a force of the biasing mechanism. Any suitable retaining member may be used.

The release member of the plunger assembly may preferably be associated with the retaining member so that the plunger may be selectively released under a force of the biasing mechanism for movement through the delivery stroke.

The retaining member may be of any suitable size, shape and construction.

For example, in some embodiments, the retaining member may include a clip, configured to be clipped about an outer end of the plunger (or an adjacent portion of the plunger rod at or near the outer end) protruding through the outer end of the barrel for retaining the plunger in the retracted position against the biasing force of the biasing mechanism. In such embodiments, the retaining member may dually function as the release member and may be simply removed or dissociated from the plunger to allow the plunger to move through the delivery stroke under the force of the biasing mechanism.

In other embodiments, the plunger may be releasably retained in the retracted position relative to the barrel by a retaining mechanism, or parts thereof. The retaining mechanism or parts thereof may or may not be of integral formation with each of the plunger and the barrel.

In some such embodiments, the retaining mechanism may include a first part associated with the plunger and a second part connectable to the first part associated with barrel.

The retaining mechanism may include mateable male and female formations that releasably couple together, such as, e.g., a threaded connection, an interference (snap-fit) connection, a bayonet-type connection or a friction fit-type connection.

In some such embodiments, the first part of the retaining mechanism associated with the plunger may include a male formation configured to be releasably inserted into, or coupled with, a female formation of the second part of the retaining mechanism associated with the barrel.

Conversely, in other such embodiments, the first part of the retaining mechanism may include a female formation configured to releasably receive, or be coupled with, a male formation of the second part of the retaining mechanism.

In preferred embodiments, the retaining mechanism may include one or more notches or grooves defined on the plunger and a key member operatively associated with the barrel and configured to be releasably received in one of the one or more notches or grooves for retaining the plunger in the retracted position against the biasing force of the biasing mechanism.

The one or more notches or grooves may be defined along a length of the plunger rod, typically along a portion at or near the outer end.

Each notch or groove may extend transversely across the plunger rod. Each notch or groove may be of a sufficient size to receive at least a portion of the key member therein.

The key member may be an elongate member including an engaging end configured to be at least partially received in one of the one or more notches or grooves defined on the plunger rod, an opposed outer end and an elongate body extending therebetween.

In use, the key member may be configured to be received through an opening defined in a sidewall of the barrel at or near the outer end such that the engaging end may (and most of the elongate body) may protrude inwards for engagement with the one or more notches or groove of the plunger rod and the outer end may protrude outwards from the sidewall of the barrel and function as the release member.

That is, in such embodiments, the outer end of the key member may be depressed or slid sideways to pivot or move the key member clear of the notch or groove thereby releasing the retaining mechanism and allowing the plunger to move through the delivery stroke.

In other embodiments, the plunger mechanism may include a separate release member operatively associated with the retaining mechanism and configured to be actuated to release the retaining mechanism.

For example, in some such embodiments, the release member may include an actuator or an actuating mechanism for releasing the retaining mechanism. For example, the actuator or actuating mechanism may include a push-button actuator, or electromechanical solenoid configured to act on the key member when actuated to release the retaining mechanism and allow the plunger to move through the delivery stroke.

As indicated, the spring-loaded plunger or the biasing mechanism may be adjustable, preferably so that an impact velocity of the plunger as it moves through the delivery stroke may be adjusted.

By way of explanation, studies have shown that increasing impact velocity of the plunger moving through the delivery stroke reduces an insertion force required to break a skin's outer layer with the microneedle patch and improves a safety margin for the microneedle patch, which is a ratio of the microneedle patch's fracture force to the insertion force (Davis, S. P. et al. 2004).

An adjustable impact velocity may be used to control a penetration depth of a microneedle device delivered by the applicator to achieve maximal penetration without stimulating an underlying nerve system of the subject (and thereby causing pain). Advantageously, this may enable the applicator to be adjusted to deliver a microneedle device to different surface (i.e., skin) thicknesses.

Generally, the adjustable spring-loaded plunger or the biasing mechanism may provide any suitable impact velocity to achieve maximal penetration without stimulating an underlying nerve system of the subject. Typically, the impact velocity may range from between about 0.5ms⁻¹ and about 100ms⁻¹, from between about 0.5ms⁻¹ and about 75.0ms⁻¹, from between about 0.5ms⁻¹ and about 50.0ms⁻¹, or from between about 0.5ms⁻¹ and about 25.0ms⁻¹.

Preferably, the adjustable spring-loaded plunger or the biasing mechanism may provide an impact velocity of at least 0.5ms⁻¹.

A person skilled in the art will appreciate that the impact velocity may be adjusted to suit an intended subject. For example, in scenarios in which the subject is a mammal a higher impact velocity may be required than in other scenarios in which the subject is a plant.

The impact velocity of the plunger may be adjustable in any suitable way.

In some embodiments the retracted position of the plunger may be lengthened or shortened to thereby adjust the impact velocity of the plunger during the delivery stroke. In such embodiments, a shorter retracted position (relative to the open delivery end of the barrel) may correspond with a reduced impact velocity.

In some such embodiments, the one or more notches or grooves defined on the plunger rod may correspond to predetermined impact velocities. Accordingly, and for example, an operator may retract the plunger to a selected notch or groove corresponding to a desired impact velocity and engage the retaining mechanism. Typically, in such embodiments, the one or more notches or grooves may further include corresponding indicia or labels indicating the corresponding impact velocity.

In other embodiments, compression of the spring when the plunger is in the retracted position may be adjustable to thereby adjust the mechanical energy stored in the spring and thereby the impact velocity of the plunger during the delivery stroke. In such embodiments, increased compression may increase the mechanical energy stored in the compressed spring, which may correspond to an increased impact velocity during the delivery stroke.

In such embodiments, the compression of the spring may preferably be adjusted independent of the plunger travel distance. For example, the plunger assembly may further include a plate member located between the outer end of the barrel and the coil spring and an actuating mechanism for moving the plate member towards and away from the outer end of the barrel to thereby adjust compression of the spring.

The plate member may be of any suitable size, shape and construction to be slidable within the barrel. Like with the plunger, the plate member may be formed from plastic and/or metal material or materials, preferably plastic.

Like the plunger, the plate member may typically be formed from a mould, such as, e.g., by compression moulding, injection moulding or blow moulding, preferably injection moulding. In such embodiments, the plate member may be formed from a polymeric material or materials.

The plate member may include a pair of opposed surfaces interconnected by opposing edges.

The opposed surfaces may include a spring-facing surface and an opposed outer surface.

The opposing edges may include at least one side edge.

As indicated, the plate member may include any suitable cross-sectional shape that is slidable within the barrel. Typically, the plate member may have a same or similar cross-sectional shape to the barrel. Preferably, the plate member may have a circular cross-sectional shape.

Likewise, the plate member may be of any suitable size sufficient to compress the spring. Generally, the plate member may substantially occupy a width dimension of the passageway of the barrel.

Any suitable actuating mechanism may be used. The actuating mechanism may preferably be manually actuated, although the use of a drive is also envisaged. Movement may be linear movement, although non-linear movement, such as, e.g., rotary movement is also envisaged.

The actuating mechanism may include one or more of a lever, an operable handle, a sliding arrangement, a hinged arrangement, or a pivoting arrangement for moving the plate member towards and away from the outer end of the barrel.

In preferred embodiments, the actuating mechanism may work together with a biasing force of the spring so that movement of the plate member away from the outer end works against the force of the spring, and so that movement of the plate member towards the outer end works under the force of the spring.

In preferred embodiments, the actuating mechanism may include a threaded arrangement extending through the outer end and operatively associated with the plate member for moving the plate member towards and away from the outer end.

The threaded arrangement may include a threaded bore defined in the outer end and an adjustment screw operatively associated with an outer surface of the plate member and configured to threadingly engage with the threaded bore for moving the plate member relative to the outer end.

The adjustment screw may have a distal end operatively associated with the plate member, an opposed head and a threaded shank extending therebetween.

The threaded shank may include an external thread extending at least partially along a length of the threaded shank. The external thread may have any suitable thread profile and ratio. For example, the external thread may have a square, triangular, trapezoidal or other profile shape. Typically, the threaded shank may have a screw thread profile with a trapezoidal outlines, preferably an Acme thread form or trapezoidal metric thread form.

The head of the adjustment screw may include an enlarged head or knob or the like to assist manual rotation of the adjustment screw by an operator. The enlarged head or knob may include knurling.

In some embodiments, the head may include at least one handle for applying torque to the adjustment screw, preferably winged handles.

In some embodiments, the shank of the adjustment screw may further include corresponding indicia or labels indicating a corresponding impact velocity achieved at selected compression of the spring.

As mentioned, the applicator includes a microneedle patch holder for releasably holding the microneedle patch through the delivery stroke and for being operatively associated with the electric actuator.

The microneedle patch holder may be of any suitable size, shape and construction.

Generally, the holder may include a plate member extending from, or mounted to, the driving end of the plunger.

The plate member may be of unitary or separate construction with the plunger, preferably the former.

The plate member may include a pair of opposed surfaces interconnected by opposing edges.

The pair of opposed surfaces may include a patch mounting surface and an opposed surface.

The opposing edges may include at least one side edge.

Preferably, the plate member may be of a size and shape configured to be slidable within the barrel during a delivery stroke. In this regard, the plate member may preferably have a size and shape complementary to the size and shape of the passageway of the barrel.

A microneedle patch may be releasably held by the holder in any suitable way, preferably to the mounting surface of the plate member.

For example, in some embodiments, the patch may be releasably fastened to the mounting surface of the plate member by one or more chemical fasteners, such as, e.g., a wet adhesive, a dry adhesive or a double-sided adhesive tape. A person skilled in the art will appreciate that any chemical fastener used will be a weak adhesive capable of enabling the patch to become detached upon completion of the delivery stroke.

In other embodiments, the patch and the mounting surface of the plate member may be fastened together by a connecting mechanism or part thereof. For example, the patch may include a first part of the connecting mechanism associated with the outer surface of the patch and the patch holder may have a second part connectable to the first part and associated with the mounting surface of the plate member.

The connecting mechanism may include mateable male and female formations that couple or mate together, including interference (snap-fit) or hook-and-loop type connections, for example.

In preferred embodiments, the patch may be releasably fastened to the mounting surface of the holder by surface tension. For example, the outer surface of the patch may be partially moistened and applied against the mounting surface of the patch holder such that it is releasably held in place by surface tension. Typically, the outer surface of the patch may be partially moistened with an aqueous solution, e.g., water.

As indicated, the applicator includes at least one leg pivotally coupled to a side of the barrel and configured to stretch taut the underlying surface, preferably at least two legs coupled to sides of the barrel, more preferably opposite sides of the barrel.

The at least one leg may stretch the underlying surface any suitable amount to draw the surface taut. For example, the at least one leg may stretch the underlying surface from between about 0.001% to about 100% relative to its original state.

Each leg may be of any suitable size, shape and construction and may be formed from any suitable material or materials. Each leg may be of unitary construction or may be formed from two or more leg pieces joined together, preferably the latter.

Generally, each leg may be formed from plastic and/or metal material or materials, preferably plastic.

Each leg may include a pair of opposed ends and an elongate body extending longitudinally therebetween.

The pair of opposed ends may include a proximal end pivotally coupled to a side of the barrel and the opposed distal end pivotable relative to the barrel and configured to contact the surface of the subject for stretching the surface taut.

The elongate body may be defined by at least one sidewall extending longitudinally between the opposed ends, preferably four sidewalls. Preferably, the elongate body may have a substantially rectangular cross-sectional shape.

The proximal end may be pivotally coupled in any suitable way and at any suitable location along a side of the barrel. Generally, the proximal end of each leg may be pivotally coupled at a height between halfway and the delivery end of the barrel.

Typically, the proximal end of each leg may be hingedly connectable to the side of the barrel by at least one hinge or part thereof.

The at least one hinge may be of any suitable size, shape and form and formed from any suitable material or materials. For example, the hinge may be a barrel hinge, a pivot hinge, a flag hinge or a living hinge.

The at least one hinge or parts thereof may or may not be of integral formation with each of the proximal end of the leg and the side of the barrel.

In some embodiments, the at least one hinge may include a first hinge part associated with the side of the barrel, a second hinge part associated with the proximal end of the leg and a pivot pin pinning the hinge parts together. In such embodiments, the distal end may be pivotable relative to the barrel about the axis of the pivot pin, preferably a horizontal axis.

The distal end may further include a foot member for contacting the surface of the subject. The foot member may orthogonally extend relative to the elongate member.

In some embodiments, the foot member may further include a gripping member for facilitating gripping of the surface. The gripping member may be a pad, liner or coating at least partially applied about the foot member.

Suitably, the gripping member may be formed from a resiliently deformable material or materials, such as, e.g., rubber or soft plastic material or materials.

As indicated, each leg may further include a biasing mechanism so that pivoting of the leg against the barrel works against the force of the biasing mechanism and so that leg pivots away from the barrel under the force of the biasing mechanism.

The biasing mechanism may include one or more springs, preferably one or more torsion springs operatively associated with the at least one hinge or parts thereof hingedly connecting each leg to a side of the barrel.

In some embodiments, each leg may be slidably mountable to a side of the barrel such that a proximal end of the leg may slide at least partially along a height of the barrel, preferably from the delivery end of the barrel at least partially towards the outer end.

In such embodiments, the barrel of the applicator may further include at least two guide tracks mountable to, or extending from, the sidewall of the barrel for guiding the respective legs in movement at least partially along the height of the barrel.

Each guide track may be substantially elongate.

Each guide track may have a substantially constant cross sectional profile.

Each guide track may have closed opposed ends. The closed opposed ends may include an upper closed end located closest the outer end of the barrel and an opposed lower closed end located at or near the delivery end of the barrel.

Each guide track may have a channel for guiding passage of the respective legs. The channel may be of any suitable cross sectional shape to facilitate movement of the legs along the channel but prevent lateral movement or separation of the legs away from the channel.

Generally, each guide track may include three portions when viewed in cross section: a first portion, a second portion and a third portion that together form the channel. Typically, the second portion bridges the first and third portions.

In some embodiments, each channel may have a substantially C- or U-shaped profile shape.

Each leg may be slidably mountable to a respective guide track, preferably indirectly via the first hinge part to which the second hinge part associated with the proximal end of the leg is pinned to by the pivot pin.

In some embodiments, each guide track may further include a biasing mechanism for biasing a proximal end of the leg towards the delivery end of the barrel. Movement of the proximal end of the leg away from the delivery end may work against a force of the biasing mechanism. Conversely, movement of the proximal end of the leg towards the delivery end may work under the force of the biasing mechanism.

The biasing mechanism may include one or more springs, preferably one or more compression springs located within each guide track between the upper closed end and the first hinge part.

Advantageously, in use the biasing members associated with the guide tracks assist in biasing the distal ends of the legs forward of the delivery end of the barrel for contact with the surface of the subject whereas the biasing members associated with the pivotal coupling of the legs to the sides of the barrel bias the legs away from the barrel to draw the surface taut underneath the open delivery end of the barrel.

In some embodiments, the applicator may include more than two legs pivotally coupled to the barrel. For example, the applicator may include three, four or even five legs spaced about a periphery of the barrel for stretching the surface of the subject taut.

As indicated, the applicator further includes an electric actuator operatively associated with the microneedle patch holder and configured to vibrate at least the microneedle patch holder at a selected frequency during the delivery stroke.

The application of ultrasonic vibrations are envisaged to enhance both the delivery and extraction of therapeutic agents and biological samples, respectively. Indeed, the vibrations are envisaged to enhance passage of the therapeutic agents and biological samples through the passageways generated by the microneedles as well as facilitating penetration of the microneedles through the surface of the substrate.

Any suitable electric actuator may be used that is capable of generating a vibratory output in response to an electrical signal input. The actuator may be electrically actuated. Preferably, the actuator may be an electromechanical device.

For example, the electric actuator may be selected from any one of an eccentric rotating mass (ERM), a linear resonant actuator (LRA), a piezo haptic actuator, a thermoelectric device, a solenoid actuator and an ultrasonic transducer or sensor.

In some embodiments, the electric actuator may include a speaker configured to emit soundwaves in response to an electrical signal input. The emitted soundwaves may generate vibrations at least in the microneedle patch holder.

In other embodiments, the electric actuator may include a piezo haptic actuator configured to generate a plurality of physical displacement strokes in response to an electrical signal input. The emitted strokes may generate vibrations at least in the microneedle patch holder.

In yet other embodiments, the electric actuator may include an eccentric rotating mass (ERM) actuator. In such embodiments, the microneedle patch holder may at least partially encompass an electric motor having a drive shaft and an unbalanced mass mounted to the drive shaft. The unbalanced mass may include a cam or the like and may be configured to generate vibrations at least in the microneedle patch holder when it is rotated by the electric motor in response to an electrical signal input.

In yet further embodiments, the electric actuator may include a linear resonant actuator ("LRA"). In such embodiments, the microneedle patch holder may at least partially enclose the LRA, which may generate vibrations at least in the microneedle patch holder in response to an electrical signal input.

In preferred embodiments, the electric actuator may include more than one actuator. For example, the microneedle patch holder may include an ERM actuator and an LRA.

The electric actuator may be adjustable to thereby adjust the frequency of vibrations generated. Generally, the electric actuator may include a controller enabling an operator to control operation of the electric actuator and/or adjust the vibrational frequency.

The electric actuator may have a vibrational frequency of between about 5 Hz and 3MHz, between about 5 Hz and about 2.5MHz, between about 5Hz and about 2MHz, between about 5Hz and about 1.5MHz, between about 5Hz and about 1MHz, between 5Hz and about 550 Hz, between about 5 Hz and about 500 Hz, between about 5 Hz and about 450 Hz, between about 5 Hz and about 400 HZ, between about 5 Hz and about 350 Hz, between about 5 Hz and about 300 Hz or between about 5 Hz and about 250 Hz.

Preferably, the electric actuator may have a vibrational frequency of at least 5 Hz.

Like with the impact velocity, a person skilled in the art will appreciate that the vibration frequency may be adjusted to suit an intended subject. For example, in scenarios in which the subject is a mammal a high vibrational frequency may be required than in other scenarios in which the subject is a plant wherein a lower vibrational frequency may be required.

The electric actuator may further include at least one power source for providing the electrical input signal, preferably via the controller.

In some embodiments, the power source may include an on-board power source, such as, e.g., one or more batteries, preferably associated with the controller.

In other embodiments, the power source may include a mains supply, preferably connectable via the controller.

The electric actuator, the controller and the power source may be electrically connected by wiring extending therebetween, preferably secured clear of the moving components of the applicator.

The controller may include one or more keys, buttons, switches and/or dials for an operator to control operation of the electric actuator.

For example, in some embodiments, the controller may include a dial enabling the operator to activate and increase/decrease the vibration frequency of the electric actuator.

In some embodiments, the applicator may further include software configured to be run on an external processing device for interacting with the controller.

The external processing device may include a computer, a tablet, a smart phone, a smart watch or a PDA, for example.

In some such embodiments, the software may include an app configured to be run on a mobile said external processing device, such as, e.g., a tablet or smart phone.

In other embodiments, the software may be a software program, browser extension and/or plug-in extension configured to be run on the external processing device, such as, e.g., a computer.

In preferred embodiments, the controller may include a button for turning the electric actuator on and off and a dial for adjusting the vibrational frequency of the electric actuator. In such embodiments, the button may be mounted on one side of the barrel and the dial may be located on an opposite side.

In some embodiments, the applicator may further include a sampler for sampling a biological sample, such as, e.g., blood, from a surface of the subject. The sampler may be of any suitable size, shape and form.

Generally, the sampler may sample a biological sample by generating negative pressure at the surface of the subject.

In some embodiments, the sampler may include a pump system configured to generate the negative pressure at the surface of the subject upon completion of the delivery stroke to facilitate the flow and collection of the biological sample.

In some such embodiments, the sampler may include an electric pump housed within the barrel of the applicator, one or more reservoirs defined on the outer surface of the microneedle patch for receiving the sample, one or more through openings extending through the microneedle patch in fluid communication with the reservoir for passage of the sample from the surface of the subject to the reservoir and a passageway defined through the microneedle patch holder and operatively connected with an electric pump for application of a negative pressure at the surface of the subject upon completion of the delivery stroke.

In use, the electric pump may be activated to generate a vacuum on the microneedle patch holder and thereby negative pressure at the surface of the subject to cause a volume of the biological sample to flow through the one or more through openings extending through the microneedle patch and into the reservoir.

Suitably, the passageway defined through the microneedle patch holder and/or the lower end of the plunger may be connected to the electric pump via a length of tubing. The passageway may include an upper opening located adjacent an upper portion of plunger and an opposed lower opening centrally located relative to the microneedle patch holder and/or the lower end of the plunger.

The outer surface of the microneedle patch may have one or more concave surfaces defining one or more reservoirs for receiving the biological sample. Likewise, the needle protruding surface of the microneedle patch may have one or more concave surfaces defined thereon.

In preferred embodiments, the one or more through openings may be associated with channels of one or more of the plurality of microneedles extending from the needle protruding surface of the patch. The channels may provide fluid communication between the one or more of the plurality of microneedles and the reservoir for extraction of the biological sample.

In some such embodiments, the microneedle patch may be attached to the microneedle patch holder with one or more releasable fasteners, such as, e.g., a bayonet-type or friction-fit connecting mechanism or an adhesive, and one or more sealing members, such as, e.g., a soft form or rubber layer extending between and about a periphery of the microneedle patch and the holder. Advantageously, use of the one or more sealing members prevents a loss of vacuum.

It is envisaged that the electric pump may be powered by the at least one power source.

It is also envisaged that the electric pump may be controlled by the controller to thereby adjust a strength of the vacuum generated and thereby the negative pressured applied at the surface of the subject.

According to a third aspect of the present invention, there is provided a method of delivering a microneedle patch to a surface of a subject with an applicator according to the first or second aspect, said method including:
positioning an open delivery end of a barrel of the applicator against the surface of the subject;
stretching taut the surface of the subject with at least one leg of the applicator;
vibrating at least a microneedle patch holder at a selected frequency with an electric actuator to facilitate penetration of the microneedle patch into the surface of the subject; and
releasing the plunger for moving the microneedle patch through a delivery stroke towards the open delivery end of the barrel of the applicator for delivery to the surface of the subject.

The method may include one or more features or characteristics of the applicator as hereinbefore described.

Optionally, the method may include an initial step of applying a microneedle patch to the microneedle patch holder.

Further, the method may include an optional step of retracting the plunger to the retracted position and activating the retaining mechanism to retain the plunger in the retracted position against a force of the biasing mechanism.

In some embodiments, the method may include adjusting the retracted position of the plunger so that the plunger provides a desired impact velocity during the delivery stroke.

In other embodiments, the method may include adjusting a compression of the spring about the plunger so that the plunger provides a desired impact velocity during the delivery stroke.

The positioning may include an initial step of squeezing the leg towards the barrel so that the distal ends extend forward of the open delivery end of the barrel.

The position may then include contacting the surface of the subject with the distal end of the leg and then pressing the open delivery end of the barrel towards the surface until the open delivery end subsequently makes contact with the surface.

The pressing of the open delivery end of the barrel towards the surface may simultaneously cause the leg to slide along it guide track against the force of the biasing mechanism towards the outer end of the barrel.

The stretching may include the pivoting of the leg away from the barrel under the force of the associated biasing mechanism to thereby cause the underlying surface to be stretched taut.

The vibrating may include activating the electric actuator associated with the microneedle patch holder to vibrate at a selected frequency.

The selected frequency may be selected based on the intended subject. The selected frequency range may range from about 5 Hz to about 3 MHz or greater. Preferably, an operator may activate and select the selected range using the controller mounted to a side of the barrel.

In some embodiments, the releasing may include depressing or sliding the outer end of the key member to pivot or move the key member clear of the notch or groove defined in the plunger rod and thereby release the retaining mechanism and allow the plunger to move through the delivery stroke.

In other embodiments, the releasing may include actuating an actuator associated with a retaining mechanism to release the retaining mechanism and allow the plunger to move through the delivery stroke.

The reference to any prior art in this specification is not and should not be taken as an acknowledgement or any form of suggestion that the prior art forms part of the common general knowledge.

### BRIEF DESCRIPTION OF DRAWINGS

Preferred features, embodiments and variations of the invention may be discerned from the following Detailed Description which provides sufficient information for those skilled in the art to perform the invention. The Detailed Description is not to be regarded as limiting the scope of the preceding Summary of Invention in any way. The Detailed Description will make reference to a number of drawings as follows:
Figures 1A and 1B are photographs respectively showing opposite sides of an applicator for delivery of a microneedle patch to a surface of a subject according to an embodiment of the present invention;
Figure 2 is a sectional side view of the applicator as shown in Figures 1A and 1B;
Figure 3 is another sectional side view of part of the applicator as shown in Figures 1A, 1B and 2;
Figure 4 is sectional side view of an applicator according to another embodiment of the present invention; and
Figure 5 is a flowchart showing steps in a method of delivering a micro needle patch to a surface of a subject using the applicator as shown in Figures 1A, 1B, 2 and 3.

### DETAILED DESCRIPTION

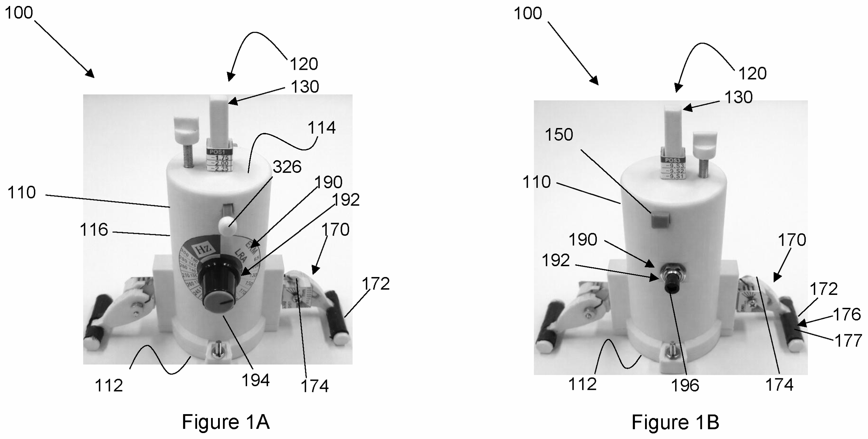

Figures 1A, 1B, 2, 3 and 4 show embodiments of an applicator (100) and parts thereof for delivery of a microneedle patch to a surface of a subject.

Referring to Figures 1A and 1B, the applicator (100) includes a barrel (110) having an open delivery end (112) through which the microneedle patch is delivered to an underlying surface of a subject; a plunger mechanism (120) including a plunger (130) slidable within the barrel (110) for moving the microneedle patch through a delivery stroke towards the open delivery end (112), an adjustable biasing mechanism (140; not visible) associated with the plunger (130) for biasing the plunger (130) towards the open delivery end (112) and a release member (150; visible only in Figure 1B) for releasing the plunger (130) under a force of the biasing mechanism (140; not visible) for movement through the delivery stroke; a microneedle patch holder (160; not visible) operatively associated with an end of the plunger (130) for releasably holding the microneedle patch through the delivery stroke; two opposed legs (170) pivotally coupled to sides of the barrel (110) and configured to stretch taut the underlying surface, each leg (170) including a distal end (172) for contacting the surface and a biasing mechanism for biasing the leg (170) away from the barrel (110) and thereby stretching the surface taut; and an electric actuator (190) operatively associated with the microneedle patch holder (160; not visible) and configured to vibrate the microneedle patch holder (160; not visible) at a selected frequency during the delivery stroke to facilitate penetration of the surface by the microneedles and enhance the passage of therapeutic agents and/or biological samples through the passageways generated by the microneedles.

The microneedle patch includes a generally planar support member and a plurality of microneedles arranged thereon and extending from a common side of the support member.

Referring to Figure 1A, the barrel (110) is of a size, shape and construction to house the plunger (130), the microneedle patch holder (160; not visible), and the electric actuator (190).

The barrel (110) includes the open delivery end (112), an opposed outer end (114) and at least one curved sidewall (116) longitudinally extending therebetween.

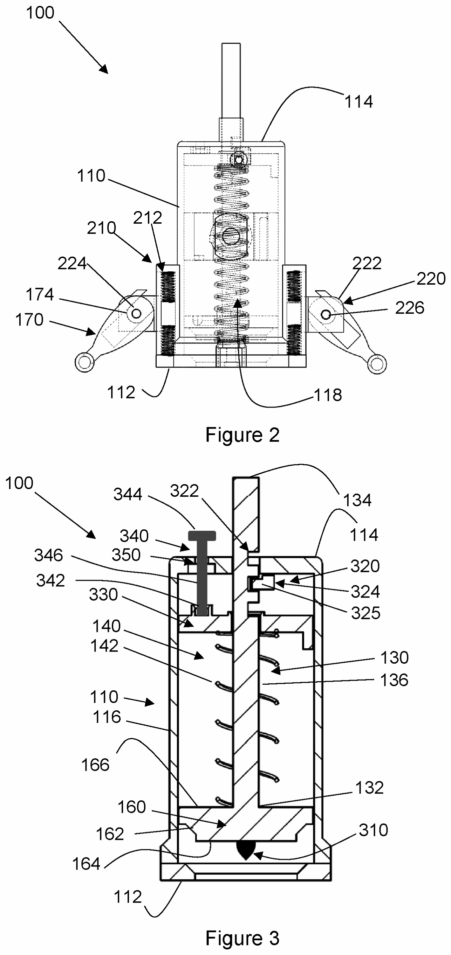

Referring briefly to Figure 2, the open delivery end (112) includes a passageway (118) defined therein for slidably receiving the plunger (130). The passageway (118) is centrally defined in the open delivery end (112). The passageway (118) extends from the open delivery end (112) at least partially towards the opposed outer end (114).

The passageway (118) has a constant diameter extending from or near the open delivery end (112) at least partially towards the opposed outer end (114).

Referring to Figure 3, and as indicated, the applicator (100) includes a plunger (130) slidable within the barrel (110) for moving the microneedle patch (310) through a delivery stroke towards the open delivery end (112).

The plunger (130) includes a pair of opposed ends an elongate body extending therebetween.

The opposed ends include a driving end (132) and an opposed outer end (134).

The microneedle patch holder (160) extends from the driving end (132) and is of unitary construction with the plunger (130).

As shown, the opposed outer end (134) extends through an opening defined in the outer end (114) of the barrel (110) so as to allow an operator to manually pull the plunger (130) towards the outer end (114) of the barrel (110) after a delivery stroke.

The elongate body is in the form of a plunger rod (136) defined by at least one curved sidewall extending longitudinally between the opposed ends.

In use, the plunger (130) is slidable within the barrel (110) between a retracted position and an extended position.

In the retracted position, the plunger (130) is manually retracted towards the outer end (114) of the barrel (110) against a force of the biasing mechanism (140).

In the extended position, the microneedle patch holder (160) is located at or near the open delivery end (112) of the barrel (110).

The biasing mechanism (140) includes a compression spring (142) configured to sit about the plunger rod (136) between the microneedle patch holder (160) and an inner surface of the outer end (114) of the barrel (110).

In use, movement of the plunger (130) to the retracted position compresses the spring (142) and stores mechanical energy configured to be released and drive the plunger (130) through the delivery stroke.

As shown, the plunger mechanism (120) further includes a retaining mechanism (320) for retaining the plunger (130) in the retracted position against a force of the biasing mechanism (140).

The retaining mechanism (320) includes one or more notches or grooves (322) defined on the plunger (130) and a key member (324) operatively associated with the barrel (110) and configured to be releasably received in one of the one or more notches or grooves (322) for retaining the plunger (130) in the retracted position against the biasing force of the biasing mechanism (140).

The one or more notches or grooves (322) defined along a length of the plunger rod (136) along a portion at or near the outer end (134).

Each notch or groove (322) extends transversely across plunger rod (136) and is of a sufficient size to receive at least a portion of the key member (324) therein.

The key member (324) is an elongate member including an engaging end (325) configured to be at least partially received in one of the one or more notches or grooves (322) defined on the plunger rod (136), an opposed outer end (326; shown in Figure 1A) and an elongate body extending therebetween.

In use, the key member (324) is configured to be received through an opening defined in the sidewall (116) of the barrel (110) at the outer end (114) such that the engaging end (325) protrudes inwards for engagement with the notches or grooves (322) of the plunger rod (136) and the outer end (326; shown in Figure 1A).

Referring to Figure 1B, the release member (150) protrudes outwards from an opposite side of the barrel (110) and is configured to be depressed to pivot or move the key member (324; not shown) clear of the notch or groove (322; not visible) thereby releasing the retaining mechanism (320; not shown) and allowing the plunger (130) to move through the delivery stroke.

Referring again to Figure 3, and as indicated, the biasing mechanism (140) including the compression spring (142) is adjustable so that an impact velocity of the plunger (130) as it moves through a delivery stroke can be adjusted.

Advantageously, studies have shown that increasing impact velocity of plunger movement through a delivery stroke reduces the insertion force required to break a surface's outer layer with a microneedle patch and improves a safety margin for the microneedle patch, which is a ratio of the microneedle patch's fracture force to the insertion force (Davis, S. P. et al. 2004).

An adjustable impact velocity may be used to control a penetration depth of a microneedle device delivered by the applicator (100) to achieve maximal penetration without stimulating an underlying nerve system of the subject (and thereby causing pain). Advantageously, this may enable the applicator (100) to be adjusted to deliver a microneedle device to different surface (i.e., skin) thicknesses.

The biasing mechanism (140) provides an impact velocity ranging between about 0.5ms⁻¹ and about 100.0ms⁻¹.

In one form, the retracted position of the plunger (130) can be lengthened or shortened to thereby adjust the impact velocity of the plunger (130) during the delivery stroke. This is achieved via the one or more notches or grooves (322) defined on the plunger rod (136) and which correspond to predetermined impact velocities. Accordingly, and for example, an operator can retract the plunger (130) to a selected notch or groove (322) corresponding to a desired impact velocity and engage the retaining mechanism (320).

In another form, compression of the spring (142) of the biasing mechanism (140) can be adjusted to thereby adjust the mechanical energy stored in the spring (142) and thereby the impact velocity of the plunger (130) during the delivery stroke. This is achieved by way of plate member (330) located between the outer end (114) of the barrel (110) and the spring (142) and an adjustment screw (340) for moving the plate member (330) towards and away from the outer end (114) to thereby adjust spring compression.

The plate member (330) is of a size, shape and construction to be slidable within the barrel (110).

The plate member (330) includes a pair of opposed surfaces interconnected by opposing edges. The plate member (330) has a circular cross-sectional shape and is of sufficient size to be able to compress the spring (142) when moved away from the outer end (114) of the barrel (110).

The adjustment screw (340) is received through a threaded bore (350) defined in the outer end (114) of the barrel (110).

The adjustment screw (340) has a distal end (342) operatively associated with the plate member (330), an opposed head (344) and a threaded shank (346) extending therebetween.

The threaded shank (346) includes an external thread extending at least partially along a length of the threaded shank (346).

The head (344) of the adjustment screw (340) includes winged handles to assist manual rotation of the adjustment screw (340) by an operator.

As mentioned, the applicator (100) includes a microneedle patch holder (160) for releasably holding a microneedle patch (310) through the delivery stroke and for being operatively associated with the electric actuator (190; not visible).

The microneedle patch holder (160) includes a plate member (162) extending from the driving end (132) of the plunger (130).

The plate member (162) includes a patch mounting surface (164) and an opposed surface (166).

The microneedle patch (310) is releasably fastened to the mounting surface (164) of the holder (160) by surface tension. Generally, an outer surface of the patch (310) is partially moistened and applied against the mounting surface (164) of the patch holder (160) such that it is releasably held in place by surface tension. Usually, the outer surface of the patch (310) is partially moistened with an aqueous solution, such as, e.g., water.

Referring to Figures 1A and 1B again, and as indicated, the applicator (100) includes two opposed legs (170) pivotally coupled to sides of the barrel (110) and configured to stretch taut an underlying surface.

Each leg (170) includes a pair of opposed ends and an elongate body extending longitudinally therebetween.

The opposed ends include a proximal end (174) pivotally coupled to a side of the barrel (110) and the opposed distal end (172) pivotable relative to the barrel (110) and configured to contact the surface of the subject for stretching the surface taut.

Referring to Figure 2, each leg (170) is pivotally coupled to a side of the barrel (110) by way of a guide track (210) enabling the proximal end (174) of the leg (170) to be slidable at least partially along a height of the barrel (110) from the delivery end (112) to a location about midway towards the outer end (114).

Each guide track (210) is substantially elongate and has a substantially constant cross sectional profile. Each guide track (210) has closed opposed ends, including an upper closed end located nearest the outer end (114) of the barrel (110) and an opposed lower closed end located at the delivery end (112) of the barrel (110).

Each guide track (210) defines a channel for guiding passage of the respective legs (170). Each channel has a substantially C-shaped profile shape.

Each leg (170) is slidably mountable to a respective guide track (210) indirectly via a hinge (220).

The hinge (220) includes a first hinge part (222) slidably mounted to the guide track (210), a second hinge part (224) forming part of the proximal end (174) of the leg (170) and a pivot pin (226) pinning the hinge parts (222, 224) together. In use, the distal end (172) is pivotable relative to the barrel (110) about the axis of the pivot pin (226).

Referring briefly to Figure 1B, the distal end (172) of each leg (170) further includes a foot member (176) for contacting the surface of the subject. The foot member (176) extends orthogonally relative to a longitudinal axis of the leg (170).

As shown, the foot member (176) further includes a gripping member (177) for facilitating gripping of the surface. The gripping member (177) includes a coating at least partially applied about the foot member (176) formed from a resiliently deformable material or materials, such as, e.g., rubber or soft plastic material or materials.

Referring back to Figure 2, each leg (170) further includes a first biasing mechanism in the form of a torsion spring so that pivoting of the leg (170) against the barrel (110) works against the force of the biasing mechanism and so that leg (170) pivots away from the barrel (110) under a force of the first biasing mechanism.

Each guide track (210) further includes a second biasing mechanism in the form of a compression spring (212) for biasing the proximal end (174) of the leg (170) towards the delivery end (112) of the barrel (110). Movement of the proximal end (174) of the leg (170) away from the delivery end (112) works against a force of the biasing compression spring (212). Conversely, movement of the proximal end (174) of the leg (170) towards the delivery end (112) works under the force of the compression spring (212).

Advantageously, in use the compression spring (212) associated with the guide tracks (210) assists in biasing the distal ends (172) of the legs (170) forward of the delivery end (112) of the barrel (110) for making contact with the surface of the subject whereas the torsion springs associated with the pivotal coupling of the legs (170) to the sides of the barrel (110) biases the distal ends (172) of the legs (170) away from the barrel (110) to draw the surface taut underneath the open delivery end (112) of the barrel (110).

Referring to both Figures 1A and 1B, the applicator (100) further includes an electric actuator (190) operatively associated with the microneedle patch holder (160) and configured to vibrate the microneedle patch holder (160) at a selected frequency during the delivery stroke or sampling.

The electric actuator (190) includes both an eccentric rotating mass (ERM) and a linear resonant actuator (LRA).

The electric actuator (190) is adjustable to thereby enable adjustment of the frequency of vibrations generated. In this regards, the electric actuator (190) includes a controller (192) enabling an operator to control operation of the electric actuator (190).

The electric actuator (190) has a vibrational frequency range of between about 5 Hz and about 3 MHz.

The electric actuator (190) further includes at least one power source in the form of one or more batteries for providing an electrical input signal via the controller (192).

Referring to Figure 1A, the controller (192) includes a dial (194) provide on one side of the barrel (110) enabling the operator to increase/decrease the vibration frequency of the electric actuator (190).

Referring to Figure 1B, the controller (192) further includes a push-button actuator (196) for turning the electric actuator (190) on and off on the other side of the barrel (110).

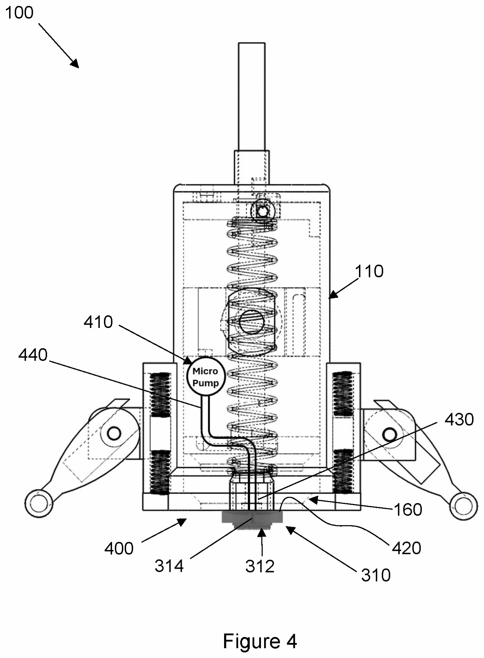

Figure 4 show the applicator (100) according to a second embodiment of the present invention. For convenience, features that are similar or correspond to feature of the earlier described embodiments will be referenced with the same reference numerals.

In this embodiment, the applicator (100) further includes a sampler (400) for obtaining a biological sample, such as, e.g., blood, from a surface of a subject.

The sampler (400) includes an electric pump (410) housed within the barrel (110), a reservoir (420) defined on an upper side of the microneedle patch (310), one or more channels (i.e., one or more through holes; 314) associated with one or more of the plurality of microneedles (312) extending from an opposed microneedle protruding surface of the microneedle patch (310) in fluid communication with the reservoir (420) for extraction of the sample from the surface of the subject to the reservoir (420) and a passageway (430) defined through the microneedle patch holder (160) and operatively connected to the electric pump (410).

In use, the electric pump (410) generates a vacuum on the microneedle patch holder (160) and thereby via the passageway (430) a negative pressure at the surface of the subject to cause the biological sample to flow through the channels (314) of the microneedles (312) and collect in the reservoir (420).

The passageway (430) defined through the microneedle patch holder (160) is connected to the electric pump (410) by a length of tubing (440). The passageway (430) includes an upper opening located adjacent an upper portion of the plunger (130) and an opposed lower opening centrally located relative to the microneedle patch holder (160).

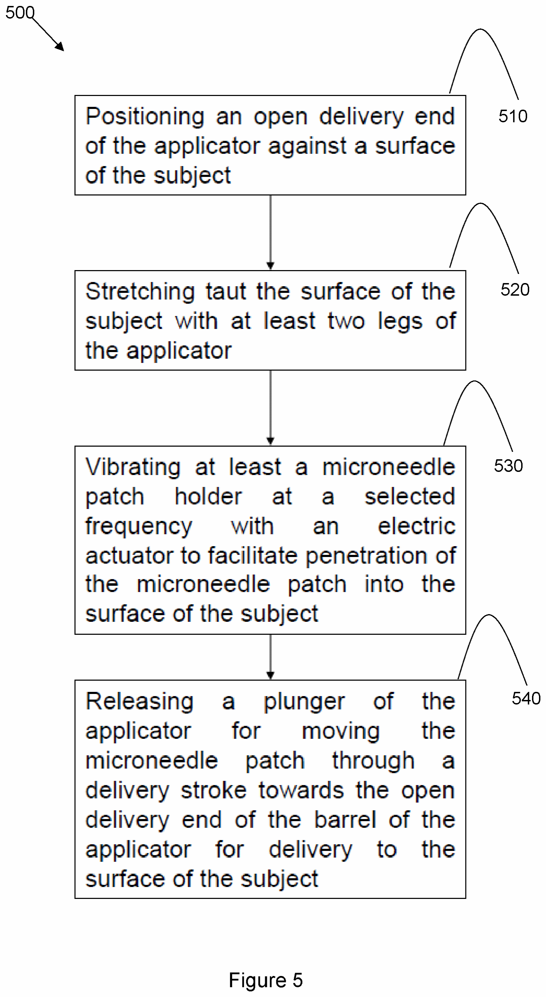

A method (500) of using the applicator (100) as shown in Figures 1A, 1B, 2 and 3 will now be described in detail with reference to Figure 5.

The method (500) optionally includes an initial step of releasably applying a microneedle patch (310) to the microneedle patch holder (160).

The method (500) optionally includes a further initial step of retracting the plunger (130) to the retracted position and activating/engaging the retaining mechanism (320) to retain the plunger (130) in the retracted position against a force of the biasing mechanism (140).

At step 510, the positioning includes an initial step of squeezing the legs (170) towards the barrel (110) so that the distal ends (172) extend forward of the delivery end (112) and then pressing the open delivery end (112) towards the surface until the end (112) makes contact with the surface.

The pressing of the open delivery end (112) of the barrel (110) towards the surface simultaneously causes the legs (170) to slide along their respective guide tracks (210) against a force of the compression spring (212).

At step 520, the stretching includes pivoting the legs (170) away from the barrel (110) under the force of the torsion springs to thereby cause the underlying surface to be stretched taut.

At step 530, the vibrating includes activating the electric actuator (190) associated with the microneedle patch holder (160) to vibrate at a selected frequency selected from about 50 Hz to about 3 MHz.

Prior to step 540, the operator can adjust the impact velocity to maximise penetration according to the surface type without stimulating the underlying nerve system of the subject. This may include altering the retracted position of the plunger (130) or the compression of the spring (142). Generally, the operator will adjust the impact velocity according to the subject and a thickness of the surface of the subject, i.e., a thicker surface will require a greater impact velocity and, conversely, a thinner surface will require a lesser impact velocity.

At step 540, the operator depresses the release member (150) to release the retaining mechanism (320) and enable the plunger (130) to move through the delivery stroke under the force of the biasing mechanism (140) for delivery of the microneedle patch (310) to the surface of the subject.

In the present specification and claims (if any), the word *'comprising'* and its derivatives including *'comprises'* and *'comprise'* include each of the stated integers but does not exclude the inclusion of one or more further integers.

Reference throughout this specification to *'one embodiment'* or *'an embodiment'* means that a particular feature, structure, or characteristic described in connection with the embodiment is included in at least one embodiment of the present invention. Thus, the appearance of the phrases *'in one embodiment'* or *'in an embodiment'* in various places throughout this specification are not necessarily all referring to the same embodiment.

### CITATIONS

Badran, M. M., Kuntsche, J., Fahr, A. Eur J. Pharm. Sci. 2009, 36, 511-523/
Davis S. P., Landis B. J., Adams Z. H., Allen M. G., Prausnitz M. R., J. Biomech. 2004, 37, 1155.
Donnelly, R. F., Garland, M. J., Morrow, D. I. J., Migalska, K., Singh, T. R. R., Majithiya, R., Woolfson, A. D., J. Controlled Release. 2010, 147, 333-341.
Ebrahiminejad, V., Prewett, P. D., Davies, G. J., Faraji Rad, Z., Adv. Mater. Interfaces. 2022, 9, 2101856.
Ma, G. & Wu, C. J. Controlled Release. 2017, 251, 11-23.
Sausse Lhernould, M., Gobillon, C., Lambert, P., ONdrugDelivery. 2013, 40, 29.

## Claims

1. An applicator (100) for delivery of a microneedle patch (310) to a surface of a subject, said applicator (100) including:
a barrel (110) having an open delivery end (112) through which the microneedle patch is delivered to an underlying said surface of the subject;
an adjustable spring-loaded plunger (130) slidable within the barrel (110) for moving the microneedle patch through a delivery stroke towards the open delivery end (112);
a microneedle patch holder (160) operatively associated with an end of the spring-loaded plunger (130) for releasably holding the microneedle patch through the delivery stroke;
at least one leg (170) pivotally coupled to a side of the barrel (110) and configured to stretch taut said underlying said surface, said leg (170) including a distal end (172) for contacting the surface and a biasing mechanism for biasing the leg (170) away from the barrel (110) and thereby stretching the surface taut; and
an electric actuator (190) operatively associated with the microneedle patch holder (160) and configured to vibrate at least the microneedle patch holder (160) at a selected frequency during the delivery stroke.

2. The applicator (100) of claim 1, wherein the adjustable spring-loaded plunger (130) is moveable between a retracted position and extended position, and, wherein, when in the retracted position, a coil spring (142) is compressed and stores mechanical energy configured to be released and drive the adjustable spring-loaded plunger (130) to the extended position as defined by the delivery stroke.

3. The applicator (100) of claim 2, wherein compression of the coil spring (142) when the adjustable spring-loaded plunger (130) is in the retracted position is adjustable to thereby adjust the mechanical energy stored in the spring (142) and thereby an impact velocity of the adjustable spring-loaded plunger (130) during the delivery stroke.

4. The applicator (100) of claim 2 or claim 3, wherein the adjustable spring-loaded plunger (130) extends through an opening defined in an outer end (114) of the barrel (110) located at an opposite end to the open delivery end (112) of the barrel (110) so as to allow an operator to manually pull the adjustable spring-loaded plunger (130) to the retracted position after a delivery stroke.

5. The applicator (100) of any one of claims 1 to 4, wherein the adjustable spring-loaded plunger (130) includes an actuating mechanism for moving a plate member (330) of the microneedle patch holder (160) towards and away from the outer end (114) of the barrel (110) to thereby adjust compression of the coil spring (142).

6. The applicator (100) of any one of claims 2 to 5, wherein the adjustable spring-loaded plunger (130) includes one or more notches or grooves (322) and a key member (324) operatively associated with the barrel (110) and configured to be releasably received in one of the one or more notches or grooves (322) for retaining the adjustable spring-loaded plunger (130) in the retracted position.

7. The applicator (100) of claim 6, wherein the key member (324) is configured to be received through an opening defined in a sidewall (116) of the barrel (110) at or near the outer end (114) such that an engaging end (325) protrudes inwards for engagement with the one or more notches or grooves (322) of the adjustable spring-loaded plunger (130) and an outer end (326) protrudes outwards from the sidewall (116) of the barrel (110) and functions as the release member (150) and wherein the one or more notches or grooves (322) defined on the spring-loaded plunger (130) correspond to predetermined impact velocities and wherein the one or more notches or grooves (322) further include corresponding indicia or labels indicating the corresponding impact velocity.

8. The applicator (100) of any one of claims 1 to 7, wherein the at least one leg (170) is hingedly connected to the side of the barrel (110) by at least one hinge (220) or part thereof and wherein the biasing mechanism includes one or more springs operatively associated with the at least one hinge (220) or part thereof for biasing the leg (170) away from the barrel (110).

9. The applicator (100) of any one of claims 1 to 8, wherein the at least one leg (170) includes a distal end (172) having a foot member (176) defined thereon and extending orthogonally relative to a remainder of the leg (170) and wherein the foot member (176) further includes a gripping member (177) for facilitating gripping of the surface.

10. The applicator (100) of any one of claims 1 to 9, wherein the at least one leg (172) is slidably mountable to the side of the barrel (110) such that a proximal end (174) of the leg (170) slides at least partially along a height of the barrel (110).

11. The applicator (100) of claim 10, wherein the barrel (110) further includes a guide track (210) mountable to, or extending from, a sidewall (116) of the barrel (110) to which the leg (170) is slidably mountable for guiding the leg (170) in movement at least partially along a height of the barrel (110) and wherein the guide track (210) includes a second biasing mechanism including one or more compression springs (212) located within the guide track (210) for in use biasing a distal end (172) of the leg (170) forward of the delivery end (112) of the barrel (110) for contact with the surface of the subject.

12. The applicator (100) of claim 8 or claim 11, wherein the biasing mechanism and the second biasing mechanism assist in biasing the distal end (172) of the leg (170) forward of the delivery end (112) of the barrel (110) for contact with the surface of the subject and in biasing the leg (170) away from the barrel (110) for drawing the surface taut underneath the open delivery end (112) of the barrel (110).

13. The applicator (100) of any one of claims 1 to 12, wherein the electric actuator (190) is adjustable to thereby adjust a frequency of vibrations generated.

14. An applicator (100) for delivery of a microneedle patch (310) to a surface of a subject, said applicator (100) including:
a barrel (110) having an open delivery end (112) through which the microneedle patch (310) is delivered to an underlying said surface of the subject;
a plunger mechanism (120) including a plunger (130) slidable within the barrel (110) for moving the microneedle patch (310) through a delivery stroke towards the open delivery end (112), an adjustable biasing mechanism (140) associated with the plunger (130) for biasing the plunger (130) towards the open delivery end (121) and a release member (150) for releasing the plunger (130) under a force of the biasing mechanism (140) for movement through the delivery stroke;
a microneedle patch holder (160) operatively associated with an end of the plunger (130) for releasably holding the microneedle patch (310) through the delivery stroke;
at least one leg (170) pivotally coupled to a side of the barrel (110) and configured to stretch taut said underlying said surface, said leg (170) including a distal end (172) for contacting the surface and a biasing mechanism for biasing the leg (170) away from the barrel (110) and thereby stretching the surface taut; and
an electric actuator (190) operatively associated with the microneedle patch holder (160) and configured to vibrate at least the microneedle patch holder (160) at a selected frequency during the delivery stroke.

15. A method of delivering a microneedle patch (310) to a surface of a subject with an applicator (100) according to any one of claims 1 to 14, said method including:
positioning an open delivery end (112) of a barrel (110) of the applicator (100) against the surface of the subject;
stretching taut the surface of the subject with at least one leg (170) of the applicator (100);
vibrating at least a microneedle patch holder (160) at a selected frequency with an electric actuator (190) to facilitate penetration of the microneedle patch (310) into the surface of the subject; and
releasing the plunger (130) for moving the microneedle patch (310) through a delivery stroke towards the open delivery end (112) of the barrel (110) of the applicator (100) for delivery to the surface of the subject.

## Patentansprüche

1. Applikator (100) für eine Abgabe eines Mikronadelpflasters (310) auf eine Oberfläche eines Subjekts, wobei der Applikator (100) einschließt:
einen Zylinder (110), der ein offenes Abgabeende (112) aufweist, durch das das Mikronadelpflaster auf eine darunterliegende Oberfläche des Subjekts abgegeben wird;
einen einstellbaren federbelasteten Kolben (130), der innerhalb des Zylinders (110) verschiebbar ist, zum Bewegen des Mikronadelpflasters durch einen Abgabehub in Richtung des offenen Abgabeendes (112);
einen Mikronadelpflasterhalter (160), der einem Ende des federbelasteten Kolbens (130) funktionell zugeordnet ist, zum lösbaren Halten des Mikronadelpflasters durch den Abgabehub;
mindestens einen Schenkel (170), der an eine Seite des Zylinders (110) schwenkbar gekoppelt und konfiguriert ist, um die darunterliegende Oberfläche straff zu spannen, wobei der Schenkel (170) ein distales Ende (172) zum Berühren der Oberfläche und einen Vorspannmechanismus zum Vorspannen des Schenkels (170) weg von dem Zylinder (110) und dadurch zum straffen Spannen der Oberfläche einschließt; und
einen elektrischen Aktuator (190), der dem Mikronadelpflasterhalter (160) funktionell zugeordnet und konfiguriert ist, um mindestens den Mikronadelpflasterhalter (160) während des Abgabehubs mit einer ausgewählten Frequenz in Schwingung zu versetzen.

2. Applikator (100) nach Anspruch 1, wobei der einstellbare federbelastete Kolben (130) zwischen einer zurückgezogenen Position und einer ausgefahrenen Position bewegbar ist, und wobei, wenn er in der zurückgezogenen Position ist, eine Schraubenfeder (142) zusammengedrückt ist und mechanische Energie speichert, die konfiguriert ist, um gelöst zu werden und den einstellbaren federbelasteten Kolben (130) in die ausgefahrene Position zu treiben, wie durch den Abgabehub definiert.

3. Applikator (100) nach Anspruch 2, wobei das Zusammendrücken der Schraubenfeder (142), wenn der einstellbare federbelastete Kolben (130) in der zurückgezogenen Position ist, einstellbar ist, um dadurch die in der Feder (142) gespeicherte mechanische Energie und dadurch eine Aufprallgeschwindigkeit des einstellbaren federbelasteten Kolbens (130) während des Abgabehubs einzustellen.

4. Applikator (100) nach Anspruch 2 oder 3, wobei sich der einstellbare federbelastete Kolben (130) durch eine Öffnung erstreckt, die in einem äußeren Ende (114) des Zylinders (110) definiert ist, das sich an einem dem offenen Abgabeende (112) des Zylinders (110) gegenüberliegenden Ende befindet, um es einem Bediener zu ermöglichen, den einstellbaren federbelasteten Kolben (130) nach einem Abgabehub manuell in die zurückgezogene Position zu ziehen.

5. Applikator (100) nach einem der Ansprüche 1 bis 4, wobei der einstellbare federbelastete Kolben (130) einen Betätigungsmechanismus zum Bewegen eines Plattenelements (330) des Mikronadelpflasterhalters (160) in Richtung des äußeren Endes (114) des Zylinders (110) und davon weg einschließt, um dadurch das Zusammendrücken der Schraubenfeder (142) einzustellen.

6. Applikator (100) nach einem der Ansprüche 2 bis 5, wobei der einstellbare federbelastete Kolben (130) eine oder mehrere Kerben oder Nuten (322) und ein Keilelement (324) einschließt, das dem Zylinder (110) funktionell zugeordnet und konfiguriert ist, um in einer der einen oder der mehreren Kerben oder Nuten (322) lösbar aufgenommen zu werden, um den einstellbaren federbelasteten Kolben (130) in der zurückgezogenen Position festzuhalten.

7. Applikator (100) nach Anspruch 6, wobei das Keilelement (324) konfiguriert ist, um durch eine Öffnung aufgenommen zu werden, die in einer Seitenwand (116) des Zylinders (110) an oder nahe dem äußeren Ende (114) derart definiert ist, dass ein Eingriffsende (325) zum Eingriff mit der einen oder den mehreren Kerben oder Nuten (322) des einstellbaren federbelasteten Kolbens (130) nach innen vorsteht und ein äußeres Ende (326) von der Seitenwand (116) des Zylinders (110) nach außen vorsteht und als das Löseelement (150) fungiert, und wobei die eine oder die mehreren Kerben oder Nuten (322), die an dem federbelasteten Kolben (130) definiert sind, vorbestimmten Aufprallgeschwindigkeiten entsprechen, und wobei die eine oder die mehreren Kerben oder Nuten (322) ferner entsprechende Markierungen oder Beschriftungen einschließen, die die entsprechende Aufprallgeschwindigkeit angeben.

8. Applikator (100) nach einem der Ansprüche 1 bis 7, wobei der mindestens eine Schenkel (170) durch mindestens ein Scharnier (220) oder einen Teil davon mit der Seite des Zylinders (110) gelenkig verbunden ist und wobei der Vorspannmechanismus eine oder mehrere Federn einschließt, die dem mindestens einen Scharnier (220) oder einem Teil davon funktionell zugeordnet sind, um den Schenkel (170) von dem Zylinder (110) weg vorzuspannen.

9. Applikator (100) nach einem der Ansprüche 1 bis 8, wobei der mindestens eine Schenkel (170) ein distales Ende (172) einschließt, das ein Fußelement (176) aufweist, das daran definiert ist und sich orthogonal relativ zu einem Rest des Schenkels (170) erstreckt, und wobei das Fußelement (176) ferner ein Greifelement (177) zum Erleichtern des Greifens der Oberfläche einschließt.

10. Applikator (100) nach einem der Ansprüche 1 bis 9, wobei der mindestens eine Schenkel (172) an der Seite des Zylinders (110) derart verschiebbar montierbar ist, dass sich ein proximales Ende (174) des Schenkels (170) mindestens teilweise entlang einer Höhe des Zylinders (110) verschiebt.

11. Applikator (100) nach Anspruch 10, wobei der Zylinder (110) ferner eine Führungsbahn (210) einschließt, die an einer Seitenwand (116) des Zylinders (110) montierbar ist oder sich von dieser erstreckt, an der der Schenkel (170) verschiebbar montierbar ist, zum Führen des Schenkels (170) in Bewegung mindestens teilweise entlang einer Höhe des Zylinders (110), und wobei die Führungsbahn (210) einen zweiten Vorspannmechanismus einschließt, der eine oder mehrere Druckfedern (212) einschließt, die sich innerhalb der Führungsbahn (210) befinden, um in Verwendung ein distales Ende (172) des Schenkels (170) vor das Abgabeende (112) des Zylinders (110) für die Berührung mit der Oberfläche des Subjekts vorzuspannen.

12. Applikator (100) nach Anspruch 8 oder 11, wobei der Vorspannmechanismus und der zweite Vorspannmechanismus dabei unterstützen, das distale Ende (172) des Schenkels (170) vor das Abgabeende (112) des Zylinders (110) für die Berührung mit der Oberfläche des Subjekts vorzuspannen und den Schenkel (170) zum straffen Dehnen der Oberfläche unter dem offenen Abgabeende (112) des Zylinders (110) von dem Zylinder (110) weg vorzuspannen.

13. Applikator (100) nach einem der Ansprüche 1 bis 12, wobei der elektrische Aktuator (190) einstellbar ist, um dadurch eine Frequenz der erzeugten Vibrationen einzustellen.

14. Applikator (100) für die Abgabe eines Mikronadelpflasters (310) auf eine Oberfläche eines Subjekts, wobei der Applikator (100) einschließt:
einen Zylinder (110), der ein offenes Abgabeende (112) aufweist, durch das das Mikronadelpflaster (310) auf eine darunterliegende Oberfläche des Subjekts abgegeben wird;
einen Kolbenmechanismus (120), der einen Kolben (130), der innerhalb des Zylinders (110) verschiebbar ist, zum Bewegen des Mikronadelpflasters (310) durch einen Abgabehub in Richtung des offenen Abgabeendes (112), einen einstellbaren Vorspannmechanismus (140), der dem Kolben (130) zugeordnet ist, zum Vorspannen des Kolbens (130) in Richtung des offenen Abgabeendes (121) und ein Löseelement (150) zum Lösen des Kolbens (130) unter einer Kraft des Vorspannmechanismus (140) für die Bewegung durch den Abgabehub einschließt;
einen Mikronadelpflasterhalter (160), der einem Ende des Kolbens (130) funktionell zugeordnet ist, zum lösbaren Halten des Mikronadelpflasters (310) durch den Abgabehub;
mindestens einen Schenkel (170), der an eine Seite des Zylinders (110) schwenkbar gekoppelt und konfiguriert ist, um die darunterliegende Oberfläche straff zu spannen, wobei der Schenkel (170) ein distales Ende (172) zum Berühren der Oberfläche und einen Vorspannmechanismus zum Vorspannen des Schenkels (170) weg von dem Zylinder (110) und dadurch zum straffen Spannen der Oberfläche einschließt; und
einen elektrischen Aktuator (190), der dem Mikronadelpflasterhalter (160) funktionell zugeordnet und konfiguriert ist, um mindestens den Mikronadelpflasterhalter (160) während des Abgabehubs mit einer ausgewählten Frequenz in Schwingung zu versetzen.

15. Verfahren zum Abgeben eines Mikronadelpflasters (310) auf eine Oberfläche eines Subjekts mit einem Applikator (100) nach einem der Ansprüche 1 bis 14, wobei das Verfahren einschließt:
Positionieren eines offenen Abgabeendes (112) eines Zylinders (110) des Applikators (100) gegen die Oberfläche des Subjekts;
Straffen der Oberfläche des Subjekts mit mindestens einem Schenkel (170) des Applikators (100);
Vibrierenlassen mindestens eines Mikronadelpflasterhalters (160) mit einer ausgewählten Frequenz durch einen elektrischen Aktuator (190), um ein Eindringen des Mikronadelpflasters (310) in die Oberfläche des Subjekts zu erleichtern; und
Lösen des Kolbens (130) zum Bewegen des Mikronadelpflasters (310) über einen Abgabehub in Richtung des offenen Abgabeendes (112) des Zylinders (110) des Applikators (100) für die Abgabe auf die Oberfläche des Subjekts.

## Revendications

1. Applicateur (100) permettant l'administration d'un timbre à micro-aiguilles (310) sur une surface d'un sujet, ledit applicateur (100) comportant :
un cylindre (110) ayant une extrémité (112) d'administration ouverte à travers laquelle le timbre à micro-aiguilles est administré sur une dite surface sous-jacente du sujet ;
un piston (130) à ressort réglable pouvant coulisser à l'intérieur du cylindre (110) pour déplacer le timbre à micro-aiguilles à travers une course d'administration vers l'extrémité (112) d'administration ouverte ;
un porte-timbre à micro-aiguilles (160) associé de manière fonctionnelle à une extrémité du piston (130) à ressort pour maintenir de manière libérable le timbre à micro-aiguilles à travers la course d'administration ;
au moins une patte (170) accouplée de manière pivotante à un côté du cylindre (110) et conçue pour tendre ladite dite surface sous-jacente, ladite patte (170) comportant une extrémité distale (172) pour venir en contact avec la surface et un mécanisme de sollicitation pour éloigner par sollicitation la patte (170) du cylindre (110) et ainsi tendre la surface ; et
un actionneur électrique (190) associé de manière fonctionnelle au porte-timbre à micro-aiguilles (160) et conçu pour faire vibrer au moins le porte-timbre à micro-aiguilles (160) à une fréquence sélectionnée pendant la course d'administration.

2. Applicateur (100) selon la revendication 1, dans lequel le piston (130) à ressort réglable est mobile entre une position rétractée et une position étendue, et, dans lequel, dans la position rétractée, un ressort hélicoïdal (142) est comprimé et stocke de l'énergie mécanique conçue pour être libérée et entraîner le piston (130) à ressort réglable à la position étendue telle que définie par la course d'administration.

3. Applicateur (100) selon la revendication 2, dans lequel la compression du ressort hélicoïdal (142) lorsque le piston à ressort réglable (130) est dans la position rétractée est réglable pour régler de ce fait l'énergie mécanique stockée dans le ressort (142) et de ce fait une vitesse d'impact du piston (130) à ressort réglable pendant la course d'administration.

4. Applicateur (100) selon la revendication 2 ou la revendication 3, dans lequel le piston (130) à ressort réglable s'étend à travers une ouverture définie dans une extrémité externe (114) du cylindre (110) située au niveau d'une extrémité opposée à l'extrémité (112) d'administration ouverte du cylindre (110) de manière à permettre à un opérateur de tirer manuellement le piston (130) à ressort réglable vers la position rétractée après une course de distribution.

5. Applicateur (100) selon l'une quelconque des revendications 1 à 4, dans lequel le piston (130) à ressort réglable comporte un mécanisme d'actionnement pour déplacer un élément plaque (330) du porte-timbre à micro-aiguilles (160) vers l'extrémité externe (114) du cylindre (110) et à l'écart de celle-ci pour régler de ce fait la compression du ressort hélicoïdal (142).

6. Applicateur (100) selon l'une quelconque des revendications 2 à 5, dans lequel le piston (130) à ressort réglable comporte une ou plusieurs encoches ou rainures (322) et un élément clé (324) associé de manière fonctionnelle au cylindre (110) et conçu pour être reçu de manière libérable dans une parmi la ou les encoches ou rainures (322) pour retenir le piston (130) à ressort réglable dans la position rétractée.

7. Applicateur (100) selon la revendication 6, dans lequel l'élément clé (324) est conçu pour être reçu à travers une ouverture définie dans une paroi latérale (116) du cylindre (110) au niveau ou à proximité de l'extrémité extérieure (114) de telle sorte qu'une extrémité de mise en prise (325) fait saillie vers l'intérieur pour une mise en prise avec la ou les encoches ou rainures (322) du piston (130) à ressort réglable et une extrémité externe (326) fait saillie vers l'extérieur depuis la paroi latérale (116) du cylindre (110) et fonctionne en tant qu'élément de libération (150) et dans lequel la ou les encoches ou rainures (322) définies sur le piston à ressort (130) correspondent à des vitesses d'impact prédéterminées et dans lequel la ou les encoches ou rainures (322) comportent en outre des indications ou étiquettes correspondantes indiquant la vitesse d'impact correspondante.

8. Applicateur (100) selon l'une quelconque des revendications 1 à 7, dans lequel l'au moins une patte (170) est reliée de manière articulée au côté du cylindre (110) par au moins une articulation (220) ou une partie de celle-ci et dans lequel le mécanisme de sollicitation comporte un ou plusieurs ressorts associés de manière fonctionnelle à l'au moins une articulation (220) ou une partie de celle-ci pour éloigner par sollicitation la patte (170) du cylindre (110).

9. Applicateur (100) selon l'une quelconque des revendications 1 à 8, dans lequel l'au moins une patte (170) comporte une extrémité distale (172) ayant un élément pied (176) défini sur celle-ci et s'étendant orthogonalement par rapport à un reste de la patte (170) et dans lequel l'élément pied (176) comporte en outre un élément de préhension (177) pour faciliter la préhension de la surface.

10. Applicateur (100) selon l'une quelconque des revendications 1 à 9, dans lequel l'au moins une patte (172) peut être montée de manière coulissante sur le côté du cylindre (110) de telle sorte qu'une extrémité proximale (174) de la patte (170) coulisse au moins partiellement le long d'une hauteur du cylindre (110).

11. Applicateur (100) selon la revendication 10, dans lequel le cylindre (110) comporte en outre une piste de guidage (210) pouvant être montée sur une paroi latérale (116) du cylindre (110) ou s'étendant à partir de celle-ci, sur laquelle la patte (170) peut être montée de manière coulissante pour guider la patte (170) en mouvement au moins partiellement le long d'une hauteur du cylindre (110) et dans lequel la piste de guidage (210) comporte un second mécanisme de sollicitation comportant un ou plusieurs ressorts de compression (212) situés au sein de la piste de guidage (210) pour, en cours d'utilisation, solliciter une extrémité distale (172) de la patte (170) en avant de l'extrémité (112) d'administration du cylindre (110) pour un contact avec la surface du sujet.

12. Applicateur (100) selon la revendication 8 ou la revendication 11, dans lequel le mécanisme de sollicitation et le second mécanisme de sollicitation aident à solliciter l'extrémité distale (172) de la patte (170) en avant de l'extrémité (112) d'administration du cylindre (110) pour un contact avec la surface du sujet et à éloigner par sollicitation la patte (170) du cylindre (110) pour tendre la surface sous l'extrémité (112) d'administration ouverte du cylindre (110).

13. Applicateur (100) selon l'une quelconque des revendications 1 à 12, dans lequel l'actionneur électrique (190) est réglable pour régler de ce fait une fréquence de vibrations générées.

14. Applicateur (100) permettant l'administration d'un timbre à micro-aiguilles (310) sur une surface d'un sujet, ledit applicateur (100) comportant :
un cylindre (110) ayant une extrémité (112) d'administration ouverte à travers laquelle le timbre à micro-aiguilles (310) est administré sur une dite surface sous-jacente du sujet ;
un mécanisme de piston (120) comportant un piston (130) pouvant coulisser au sein du cylindre (110) pour déplacer le timbre à micro-aiguilles (310) à travers une course d'administration vers l'extrémité (112) d'administration ouverte, un mécanisme de sollicitation (140) réglable associé au piston (130) pour solliciter le piston (130) vers l'extrémité (121) d'administration ouverte et un élément de libération (150) pour libérer le piston (130) sous une force du mécanisme de sollicitation (140) pour un mouvement à travers la course d'administration ;
un porte-timbre à micro-aiguilles (160) associé de manière fonctionnelle à une extrémité du piston (130) pour maintenir de manière libérable le timbre à micro-aiguilles (310) à travers la course d'administration ;
au moins une patte (170) accouplée de manière pivotante à un côté du cylindre (110) et conçue pour tendre ladite dite surface sous-jacente, ladite patte (170) comportant une extrémité distale (172) pour venir en contact avec la surface et un mécanisme de sollicitation pour éloigner par sollicitation la patte (170) du cylindre (110) et ainsi tendre la surface ; et
un actionneur électrique (190) associé de manière fonctionnelle au porte-timbre à micro-aiguilles (160) et conçu pour faire vibrer au moins le porte-timbre à micro-aiguilles (160) à une fréquence sélectionnée pendant la course d'administration.

15. Procédé d'administration d'un timbre à micro-aiguilles (310) sur une surface d'un sujet avec un applicateur (100) selon l'une quelconque des revendications 1 à 14, ledit procédé comportant :
le positionnement d'une extrémité (112) d'administration ouverte d'un cylindre (110) de l'applicateur (100) contre la surface du sujet ;
le fait de tendre la surface du sujet avec au moins une patte (170) de l'applicateur (100) ;
la vibration d'au moins un porte-timbre à micro-aiguilles (160) à une fréquence sélectionnée avec un actionneur électrique (190) pour faciliter la pénétration du timbre à micro-aiguilles (310) dans la surface du sujet ; et
la libération du piston (130) pour déplacer le timbre à micro-aiguilles (310) à travers une course d'administration vers l'extrémité (112) d'administration ouverte du cylindre (110) de l'applicateur (100) pour administrer sur la surface du sujet.
